# EUROPÄISCHE PATENTSCHRIFT

(11) **EP 0 995 735 B1**
(45) Veröffentlichungstag und Bekanntmachung des Hinweises auf die Patenterteilung: **18.12.2002**
(21) Anmeldenummer: 99117121.6
(22) Anmeldetag: 31.08.1999
(51) Int. Cl.: C07C 29/52, C07C 35/205

(54) **Verfahren zur Herstellung cyclischer Alkohole**
Process for the preparation of cyclic alcohols
Procédé de préparation d'alcools cycliques

(30) Priorität: 22.10.1998 DE 19848730
(43) Veröffentlichungstag der Anmeldung: 26.04.2000
(73) Patentinhaber: Degussa AG, 40474 Düsseldorf (DE)
(72) Erfinder: Grund, Gerda, Dr., 48249 Dülmen (DE); Günzel, Bernd, Dr., 45721 Haltern (DE)

(56) Entgegenhaltungen:
- BE-A- 668 681
- FR-A- 1 486 391
- FR-A- 1 549 178
- GB-A- 1 035 624
- US-A- 3 488 740

## Beschreibung

Die Erfindung betrifft ein neues Verfahren zur Herstellung von cyclischen Alkoholen durch Oxidation von Cycloalkanen mit 9 bis 16 C-Atomen mit Hilfe von Sauerstoff enthaltenden Gasen in Gegenwart von Borsäure.

Cyclische Alkohole großer Ringe sind wertvolle Zwischenprodukte bei der Herstellung von Riechstoffen, Pharmaka, Agrochemikalien sowie für die Herstellung von Vorprodukten von Polymeren.

Die Oxidation von Paraffinen durch Sauerstoff in Gegenwart von Borsäure ist seit langem bekannt und wird beispielsweise in DRP 552 886 (1928) beschrieben. Die Oxidation bleibt dabei überwiegend auf der Stufe des Alkohols stehen, was damit erklärt wird, daß die Borsäure den Alkohol durch Esterbildung abfängt und ihn dadurch der weiteren Oxidation zum Keton und ggf. zur Carbonsäure entzieht.

Nach H. Kalenda, Ullmanns Encyklopädie der technischen Chemie, Band 9, 1975, S. 674 wird Cyclododecanol durch Oxidation von Cyclododecan mit Borsäure hergestellt, wobei als Folgeprodukt der Oxidation unter anderem auch Cyclododecanon entsteht.

F. Broich und H. Grasemann, Erdöl-Kohle-Erdgas-Petrochemie 18, 1965, S. 360-364, beschäftigen sich mit dem Reaktionsmechanismus der Luftoxidation von cyclischen Kohlenwasserstoffen. Auch hier wird die Oxidation von Cyclododecan in Gegenwart von Borsäure beschrieben.

In Ullmanns Encyklopädie der technischen Chemie, Ergänzungsband, 1970, S. 170-177, wird bei der Diskussion der Oxidation gesättigter Kohlenwasserstoffe mit Luft auch die Oxidation von Cyclohexan zu Cyclohexanol und Cyclohexanon beschrieben. Hier wird sowohl die Oxidation in Gegenwart von Kobalt als auch von Borsäure angegeben. In Anbetracht der höheren Energie- und Investitionskosten beim Borsäure-Verfahren hat sich jedoch das Kobalt-Verfahren bei der Oxidation von Cyclohexan durchgesetzt.

Bei der Oxidation von Cyclododecan zu Cyclododecanol und Cyclododecanon wird dagegen nur Borsäure angewandt, weil man damit Ausbeuten an Cyclododecanol-Cyclododecanon-Gemischen von 75 bis 80 % auch im Umsatzbereich von 25 bis 30 % erreichte.

Die Oxidation kleiner Ringe wird dagegen mit Hilfe von Kobaltkatalysatoren durchgeführt. So wird beispielsweise in US 4 263 453 angegeben, daß man Cyclohexan mit Hilfe von zum Beispiel Kobaltacetat zu Adipinsäure oxidieren kann.

Nach EP-A-0 519 569 werden vorzugsweise Cycloalkane mit 3 bis 8 C-Atomen an Kobalt(II)-lonen enthaltendem Molekularsieb oxidiert. Aus Cyclohexan entstehen dabei überwiegend Cyclohexanol, Cyclohexanon und Adipinsäure.

Bei der Oxidation von Cyclohexan zu Cyclohexanol und Cyclohexanon wird nach US 5 767 320 ein fester Phthalocyanin- oder Porphyrinkomplex eines Übergangsmetalles als Katalysator verwendet. Dabei setzt man in den Beispielen auch Komplexe des Kobalts ein. Das Verfahren ist jedoch auf Cyclohexan beschränkt und wird ohne Borsäure durchgeführt.

Aufgabe der vorliegenden Erfindung war es, die Reaktionsgeschwindigkeit der Oxidation von großen Ringen mit 9 bis 16 C-Atomen an Borsäure zu cyclischen Alkoholen zu steigern, ohne dabei die Selektivität der Reaktion zu verschlechtern.

Die Aufgabe wird erfindungsgemäß dadurch gelöst, daß man das Cycloalkan sowie 0,2 bis 5 Gew.-% Borsäure, bezogen auf das Cycloalkan, vorlegt, während der Reaktion so viel Borsäure zudosiert, daß das Molverhältnis von gebildetem cyclischen Alkohol zur Borsäure am Ende der Reaktion bei 1: 0,6 bis 1 : 1,7 liegt, und die Reaktion außerdem in Gegenwart von 0,05 bis 5 Gew.-% Kobalt (II), bezogen auf das ursprünglich vorgelegte Cycloalkan, vornimmt.

Überraschenderweise führt die Dosierung der Borsäure und der Zusatz von Kobalt (II) zu einer deutlichen Erhöhung der Reaktionsgeschwindigkeit der Oxidation.

Bei der Oxidationsreaktion stellt man vorzugsweise ein Molverhältnis von gebildetem cyclischen Alkohol zur Borsäure von 1 : 0,8 bis 1 : 1,4 ein, wobei etwa äquimolare Mengen ganz besonders günstig sind. Während der Reaktion wird die Borsäure vorzugsweise in 1 bis 10 Portionen zugegeben, wobei die Borsäure auch kontinuierlich zudosiert werden kann.

Für die Oxidation verwendet man vorzugsweise α-Metaborsäure oder eine α-Metaborsäure bildende Borsäure, wie zum Beispiel Orthoborsäure, die bei den Oxidationstemperaturen zu α-Metaborsäure entwässert wird.

Die Konzentration von Kobalt (II) liegt vorzugsweise bei 0,1 bis 2 Gew.-%, bezogen auf die Cycloalkan-Menge am Anfang der Reaktion. Dabei bezieht man sich auf das Kobaltion und nicht auf das Kobaltsalz. Kobalt (II) wird im allgemeinen als ein organisches oder anorganisches Salz verwendet. Vorzugsweise werden Salze von Carbonsäuren mit 2 bis 18 C-Atomen eingesetzt. Beispiele dafür sind das Acetat, Oxalat, Dodecanoat, Palmitat oder Stearat.

Die oxidierenden Gase enthalten vorzugsweise 10 bis 100 % Sauerstoff, wobei Luft als Oxidationsmittel aus Kosten- und Sicherheitsgründen ganz besonders bevorzugt wird.

Beispiele für große Ringe sind Cyclononan, Cyclodecan, Cyclododecan, Cyclotridecan und Cyclohexadecan. Vorzugsweise werden Cycloalkane mit 10 bis 14 C-Atomen verwendet.

Das genannte Oxidationsverfahren liefert im wesentlichen ein Reaktionsgemisch aus Cycloalkanol-Borsäureester, Cycloalkanon und Cycloalkan. In einem weiteren Verfahrensschritt wird das Reaktionsprodukt einer Hydrolyse mit Wasser bei erhöhten Temperaturen unterworfen. Das dabei anfallende Produkt zerfällt beim Abkühlen in 2 Phasen, in eine organische Phase mit dem Cycloalkanol, dem Cycloalkanon und dem Cycloalkan und eine wäßrige Phase mit der Borsäure und dem Kobalt. Die organische Phase ist kobaltfrei.

Aus der wäßrigen Phase kann Borsäure durch Kristallisation abgetrennt werden, worauf Borsäure und Kobalt in den Oxidationsprozeß zurückgeführt werden können.

Die folgenden Beispiele sollen die Erfindung verdeutlichen.

### Vergleichsbeispiel A

In einem semikontinuierlich betriebenen 6-I-Rührkesselreaktor werden 4,3 kg Cyclododecan (CDAN), das mit Alkylperoxiden angereichert ist (ca. 75 ppm aktiver Sauerstoff), vorgelegt und auf 145 °C aufgeheizt. Dann werden 121 g Orthoborsäure zugegeben, worauf durch Luft-Dosierung von 600 N l/h die Oxidation eingeleitet wird. Die Oxidationszeit beträgt 90 Minuten.

| | |
|---|---|
| CDAN-Umsatz | 2,8% |
| Ausbeute an Cyclododecanol-Cyclododecanon-Gemisch | 2,7 % |
| Selektivität bezogen auf das Cyclododecanol-Cyclododecanon-Gemisch | 96,4% |

### Beispiel 1

Es wird wie in Vergleichsbeispiel A verfahren. Es werden jedoch 3,84 kg CDAN vorgelegt und bei Beginn der Oxidation 85,5 g Orthoborsäure und 40,5 g Kobalt-(II)-acetat zugesetzt. Nach 60 Minuten werden 36,3 g Orthoborsäure zudosiert. Die Reaktionszeit beträgt insgesamt 90 Minuten.

| | |
|---|---|
| CDAN-Umsatz | 9% |
| Ausbeute an Cyclododecanol-Cyclododecanon-Gemisch | 8 % |
| Selektivität bezogen auf das Gemisch | 89 % |

Vergleichsbeispiel A und Beispiel 1 zeigen, daß der Zusatz des Kobaltsalzes und die Dosierung der Borsäure zu einer mehrfachen Steigerung der Oxidationsgeschwindigkeit bei konstanter Temperatur von 145 °C führen.

### Vergleichsbeispiel B

In einem 6-I-Rührkesselreaktor werden wie in Vergleichsbeispiel A 4,3 kg CDAN vorgelegt und auf 145 °C aufgeheizt. Nach Zugabe von 119 g Orthoborsäure und Luft-Dosierung von 600 Nl/h wird die Oxidation eingeleitet. Nach 60 Minuten werden 51 g und nach 185 Minuten 40 g Orthoborsäure zugegeben, so daß insgesamt 210 g Orthoborsäure zugesetzt sind. Die Oxidationszeit beträgt insgesamt 300 Minuten.

| | |
|---|---|
| CDAN-Umsatz | 10,4% |
| Ausbeute an Cyclododecanol-Cyclododecanon-Gemisch | 9,5 % |
| Selektivität bezogen auf das Gemisch | 90,6 % |

### Beispiel 2

Es wird wie in Beispiel 1 verfahren. Die Gesamtreaktionszeit beträgt jedoch 180 Minuten. Außerdem werden nach 90 Minuten noch 70 g Orthoborsäure zugesetzt, so daß insgesamt 191,8 g Orthoborsäure zugegeben sind.

| | |
|---|---|
| CDAN-Umsatz | 22,6% |
| Ausbeute an Cyclododecanol-Cyclododecanon-Gemisch | 18,4 % |
| Selektivität bezogen auf das Gemisch | 81,4 % |

Vergleichsbeispiel B und Beispiel 2 zeigen, daß das erfindungsgemäße Verfahren einerseits zu einer deutlichen Steigerung der Reaktorleistung und andererseits auch im Bereich hoher Umsätze zu sehr guten Ausbeuten an Wertprodukten führt.

### Vergleichsbeispiel C

Es wird wie in Vergleichsbeispiel B verfahren. Die Reaktionstemperatur beträgt jedoch 155 °C und die Reaktionszeit 180 Minuten. Außerdem werden 118,3 g Orthoborsäure vorgelegt und während der Reaktion nach 30 Minuten 52,5 g und nach 90 Minuten weitere 39,2 g Orthoborsäure dosiert, so daß insgesamt 210 g Orthoborsäure zugegeben sind.

| | |
|---|---|
| CDAN-Umsatz | 16,6% |
| Ausbeute an Cyclododecanol-Cyclododecanon-Gemisch | 14,0 % |
| Selektivität bezogen auf das Gemisch | 84,2 % |

Vergleichsbeispiel C und Beispiel 2 zeigen, daß das erfindungsgemäße Verfahren trotz niedrigerer Temperatur zu einem höheren Umsatz führt, wodurch die Reaktorleistung gesteigert wird.

### Beispiel 3

### Aufarbeitung des Oxidats von Beispiel 1:

Das Oxidat wird nach der Reaktion mit 5 I Wasser von 50 °C versetzt, wobei die Borsäureester hydrolysieren. Die Phasen werden getrennt.

Die wäßrige Phase enthält die Borsäure und > 98 % des in der Oxidation eingesetzten Kobalts.

Die organische Phase besteht aus CDAN, Cyclododecanol, Cyclododecanon und Nebenprodukten der Oxidation. Der Kobaltgehalt liegt bei 16 wppm Co. Die organische Phase wird erneut mit 2 l Wasser gewaschen. Danach ist kein Kobalt mehr nachweisbar (< 1 wppm Co).

Die erfindungsgemäßen Beispiele belegen, daß cyclische Alkohole bei erhöhter Reaktionsgeschwindigkeit und dadurch verbesserten Umsätzen hergestellt werden. Die Qualität der Produkte ist unverändert, da das zusätzlich verwendete Kobalt quantitativ abgetrennt wird.

## Patentansprüche

1. Verfahren zur Oxidation von Cycloalkanen mit 9 bis 16 C-Atomen zu cyclischen Alkoholen mit Hilfe von Sauerstoff enthaltenden Gasen in Gegenwart von Borsäure,
**dadurch gekennzeichnet,**
**daß** man das Cycloalkan und 0,2 bis 5 Gew.-% Borsäure, bezogen auf das Cycloalkan, vorlegt, während der Reaktion so viel Borsäure zudosiert, daß das Molverhältnis von gebildetem cyclischen Alkohol zur Borsäure am Ende der Reaktion bei 1 : 0,6 bis 1 : 1,7 liegt, und außerdem die Reaktion in Gegenwart von 0,05 bis 5 Gew.-% Kobalt(II), bezogen auf das ursprünglich eingesetzte Cycloalkan, durchführt.

2. Verfahren nach Anspruch 1,
**dadurch gekennzeichnet,**
**daß** das Molverhältnis von cyclischem Alkohol zur Borsäure bei 1 : 0,8 bis 1 : 1,4 liegt.

3. Verfahren nach Anspruch 1,
**dadurch gekennzeichnet,**
**daß** die Borsäure während der Reaktion in 1 bis 10 Portionen zugesetzt wird.

4. Verfahren nach Anspruch 1,
**dadurch gekennzeichnet,**
**daß** die Borsäure während der Reaktion kontinuierlich zudosiert wird.

5. Verfahren nach Anspruch 1,
**dadurch gekennzeichnet,**
**daß** man eine α-Metaborsäure bildende Borsäure oder α-Metaborsäure selber einsetzt.

6. Verfahren nach Anspruch 1,
**dadurch gekennzeichnet,**
**daß** die Konzentration von Kobalt (II) bei 0,1 bis 2 Gew.-% liegt.

7. Verfahren nach Anspruch 1,
**dadurch gekennzeichnet,**
**daß** man Kobalt (II) in Form eines Salzes einer Carbonsäure mit 2 bis 18 C-Atomen verwendet.

8. Verfahren nach Anspruch 1,
**dadurch gekennzeichnet,**
**daß** mit Luft oxidiert wird.

9. Verfahren nach Anspruch 1,
**dadurch gekennzeichnet,**
**daß** die Cycloalkane 10 bis 14 C-Atome aufweisen.

10. Verfahren nach Anspruch 1,
**dadurch gekennzeichnet,**
**daß** man nach der Oxidation in einem Hydrolyseschritt das Kobalt (II) zusammen mit der Borsäure abtrennt.

## Claims

1. A process for the oxidation of a cycloalkane having from 9 to 16 carbon atoms to a cyclic alcohol using an oxygen-containing gas in the presence of boric acid, **characterized in that** the cycloalkane and from 0.2 to 5% by weight of boric acid, based on the cycloalkane, are charged, sufficient boric acid is added during the reaction so that the molar ratio of cyclic alcohol formed to the boric acid at the end of the reaction is from 1:0.6 to 1:1.7 and, furthermore, the reaction is performed in the presence of from 0.05 to 5% by weight of cobalt(II), based on the cycloalkane originally charged.

2. A process according to claim 1, **characterized in that** the molar ratio of cyclic alcohol to boric acid is from 1:0.8 to 1:1.4.

3. A process according to claim 1, **characterized in that** the boric acid is added in from 1 to 10 portions during the reaction.

4. A process according to claim 1, **characterized in that** the boric acid is added continuously during the reaction.

5. A process according to claim 1, **characterized in that** use is made of an α-metaboric acid-forming boric acid or α-metaboric acid itself.

6. A process according to claim 1, **characterized in that** the concentration of cobalt(II) is from 0.1 to 2% by weight.

7. A process according to claim 1, **characterized in that** use is made of cobalt(II) in the form of a salt of a carboxylic acid having from 2 to 18 carbon atoms.

8. A process according to claim 1, **characterized in that** air is used for the oxidation.

9. A process according to claim 1, **characterized in that** the cycloalkane has from 10 to 14 carbon atoms.

10. A process according to claim 1, **characterized in that**, after the oxidation, the cobalt(II) is separated off together with the boric acid in a hydrolysis step.

## Revendications

1. Procédé d'oxydation de cycloalkanes ayant de 9 à 16 atomes de carbone, en alcools cycliques à l'aide de gaz contenant de l'oxygène en présence d'acide borique,
**caractérisé en ce qu'**
on introduit le cycloalkane et de 0,2 à 5% en poids d'acide borique rapporté au cycloalkane, ajouté par doses pendant la réaction, autant d'acide borique que le rapport molaire de l'alcool cyclique formé pour l'acide borique à la fin de la réaction se situe à 1:0,6 à 1 :1,7 et en outre on effectue la réaction en présence de 0,05 à 5% en poids de Cobalt (II) rapporté au cycloalkane mis en oeuvre originellement.

2. Procédé selon la revendication 1,
**caractérisé en ce que**
le rapport molaire de l'alcool cyclique à l'acide borique se situe à 1:0,8 à 1:1,4.

3. Procédé selon la revendication 1,
**caractérisé en ce que**
l'acide borique est ajouté pendant la réaction en portions de 1 à 10.

4. Procédé selon la revendication 1,
**caractérisé en ce que**
l'acide borique est ajouté par doses pendant la réaction d'une manière continue.

5. Procédé selon la revendication 1,
**caractérisé en ce qu'**
on met en oeuvre un acide borique qui forme de l'acide α-métaborique, ou l'acide α-métaborique lui-même.

6. Procédé selon la revendication 1,
**caractérisé en ce que**
la concentration du cobalt-(II) se situe à 0,1 à 2% en poids.

7. Procédé selon la revendication 1,
**caractérisé en ce qu'**
on utilise le cobalt-(II) sous forme d'un sel d'un acide carboxylique ayant de 2 à 18 atomes de carbone.

8. Procédé selon la revendication 1,
**caractérisé en ce qu'**
on oxyde avec de l'air.

9. Procédé selon la revendication 1,
**caractérisé en ce que**
les cycloalkanes possèdent de 10 à 14 atomes de carbone.

10. Procédé selon la revendication 1,
**caractérisé en ce qu'**
on sépare après l'oxydation dans une étape d'hydrolyse, le cobalt-(II) conjointement avec l'acide borique.
